(19) Europäisches Patentamt
European Patent Office
Office européen des brevets

(11) Numéro de publication: **0 224 401 B1**

(12) **FASCICULE DE BREVET EUROPEEN**

(45) Date de publication du fascicule du brevet: **17.01.90**

(51) Int. Cl.[4]: **C07C 59/52**, C07C 59/56, C07C 59/64, C07C 51/347, C07D 333/24, C07D 317/60

(21) Numéro de dépôt: **86402337.9**

(22) Date de dépôt: **17.10.86**

(54) **Procédé d'obtention d'acides alkanoiques.**

(30) Priorité: **21.10.85 FR 8515573**

(43) Date de publication de la demande: **03.06.87 Bulletin 87/23**

(45) Mention de la délivrance du brevet: **17.01.90 Bulletin 90/3**

(84) Etats contractants désignés: **AT BE CH DE ES FR GB IT LI LU NL SE**

(56) Documents cités:
**EP-A- 0 124 407**
**FR-A- 2 415 109**
**FR-A- 2 470 127**
**GB-A- 2 067 988**

**L.F. FIESER et al.: "Lehrbuch der organischen Chemie", 3ème édition, 1957, pages 770,771, Verlag Chemie GmbH, Weinheim, DE;**

(73) Titulaire: **SOCIETE FRANCAISE HOECHST Société anonyme dite:, 3, avenue du Général de Gaulle, F-92800 Puteaux(FR)**

(72) Inventeur: **Vallejos, Jean-Claude, 9 Rue Labat, F-75018 Paris(FR)**
Inventeur: **Christidis, Yani, 53 Boulevard de la Villette, F-75019 Paris(FR)**

(74) Mandataire: **Rinuy, Santarelli, 14, avenue de la Grande Armée, F-75017 Paris(FR)**

**EP 0 224 401 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé de fabrication industrielle d'acides alkanoïques, plus particulièrement elle se rapporte à un procédé de fabrication d'acides acétiques substitués, ci-après désignés acides arylacétiques, de formule générale I,

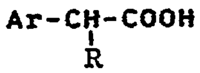

I

dans laquelle R représente un atome d'hydrogène ou un radical alkyle en $C_1$-$C_4$ et Ar représente un radical à caractère aromatique choisi parmi les radicaux suivants: thiényle-2, (méthoxy-2 naphtyle)-1, méthylènedioxy-3,4-phényle et les phényles substitués de formule générale II,

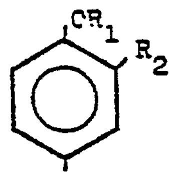

II

où $R_1$ représente un atome d'hydrogène ou un groupement alkyle en $C_1$-$C_4$ et $R_2$ représente un atome d'hydrogène, un atome d'halogène, un groupement alkyle, alcoxyle ou hydroxyle. Les acides arylacétiques de formule générale I sont largement décrits dans la littérature. Ce sont notamment des matières premières intéressantes pour la synthèse de substances pharmaceutiques actives.

Il est donc d'un grand intérêt de pouvoir les préparer d'une manière aussi rationnelle que possible.

On connait de très nombreuses méthodes pour préparer des acides acétiques substitués. Parmi celles-là, la réaction de Willgerodt-Kindler qui autorise la transformation d'une alkylarylcétone en acide arylalkylcarboxylique est l'une des plus connues. Toutefois, elle ne fournit généralement que des rendements modérés à médiocres et elle conduit à des produits secondaires soufrés responsables d'une pollution inacceptable aujourd'hui. Pour remédier à ces inconvénients, on s'est adressé à des méthodes plus élaborées nécessitant l'emploi de plusieurs stades réactionnels telles que notamment l'hydrogènolyse soit chimique, soit catalytique, d'acides arylglycoliques, d'acides o-acylé arylglycoliques ou d'acides aryl-2 halogéno-2 alkanoïques, issus dans certains cas de la condensation de l'acide glyoxylique sur le dérivé aromatique correspondant (demandes de brevet européen No. 0 032 374 et 0 028 375, brevet européen 0 003 825), l'hydrolyse d'un arylalkylacétonitrile obtenu par réaction de l'ion cyanure sur une benzylamine quaternisée convenablement substituée (demande de brevet européen No. 0 062 440). Ces méthodes, dont certaines sont très récentes, exigent comme matière première de départ des produits rarement disponibles sur le marché, ce qui nécessite leur préparation préalable avec les inconvénients que cela comporte.

On a également proposé de préparer des acides arylacétiques par réaction à chaud en milieu acide en présence de phosphore rouge et de quantités catalytiques d'iode ou d'acide iodhydrique, d'un acide carboxylique alpha carbonylé sur un hydrocarbure aromatique convenable (demande de brevet français No. 8 307 137). L'emploi de phosphore rouge et le fait que la réaction s'effectue en milieu hétérogène sont des contraintes pesantes entraînant des servitudes et des précautions de sécurité contraignantes, donc onéreuses.

L.F. Fieser et al "Lehrbuch der Organischen Chemie", 3ème édition, 1957, pages 770–771, Verlag Chemie GmbH, les brevets EP-A 0 124 407, FR-A 2 470 127, FR-A 2 415 109 et EP-A 0 032 374 décrivent également la préparation d'acides aryle, notamment phényl- ou thiényl-acétique, en phase hétérogène par action de phosphore rouge.

Enfin, le document GB-A 2 067 988 décrit la préparation d'acide thiényl-2 acétique par hydrogènation catalytique.

Or, la Demanderesse a découvert avec étonnement qu'il était possible d'obtenir économiquement et avec de bons rendements et en un seul stade réalisé en phase homogène, des acides arylacétiques de formule générale I par réaction à chaud en présence d'acide phosphoreux et de quantités catalytiques d'iode ou d'acide iodhydrique, d'un acide carboxylique alpha carbonylé de formule générale III. R–CO–COOH, dans laquelle R a la signification donnée précédemment avec un dérivé insaturé à caractère aromatique choisi parmi les suivants : thiophène, méthoxy-2 naphtalène, méthylènedioxy-1,2 benzène ou benzodioxole-1,3 et les hydrocarbures aromatiques substitués de formule générale IV, dans laquelle $R_1$ et $R_2$ ont la signification donnée précédemment :

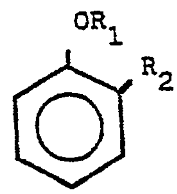

IV

Par la suite, cet ensemble de produits insaturés à caractère aromatique sera désigné A.

Plus particulièrement, le procédé selon la présente invention consiste à faire réagir à une température supérieure à 70°C en milieu acide, une mole d'un acide carboxylique alpha carbonylé, de formule générale III, avec de une à dix moles d'un dérivé appartenant à l'ensemble A en présence d'au moins un équivalent molaire d'acide phosphoreux et de 0,01 à 0,1 mole d'iode ou d'acide iodhydrique, éventuellement au sein d'un solvant organique compatible tel que notamment l'acide acétique, puis à isoler l'acide cherché de formule générale I par des moyens connus en soi.

Avantageusement, le procédé de la présente invention consiste à faire réagir au reflux du milieu réactionnel une mole d'un acide carboxylique alpha carbonylé de formule générale III avec de une à dix moles d'un dérivé appartenant à l'ensemble A en

présence d'une mole d'acide phosphoreux et de 0,05 mole d'iode ou d'acide iodhydrique puis à isoler l'acide cherché par des moyens connus en soi.

L'acide iodhydrique est l'acide iodhydrique commercial en solution aqueuse à 57 % en poids.

L'acide carboxylique alpha carbonylé est notamment l'acide glyoxylique, l'acide pyruvique, l'acide méthyl-3 oxo-2 butanoïque, l'acide oxo-2 butanoïque, l'acide triméthylpyruvique.

En fin de réaction, l'acide arylacétique cherché de formule générale I est isolé du milieu réactionnel par des moyens connus en soi. Généralement, après élimination des solvants réactionnels et neutralisation des acides minéraux présents par un sel d'acide faible tel que l'acétate de sodium, on reprend le milieu réactionnel avec de l'eau puis on élimine le dérivé aromatique non transformé par des moyens connus en soi puis on isole l'acide cherché soit par cristallisation, soit par tout autre moyen connu en soi.

Avec certains substrats, il est parfois avantageux d'isoler l'acide arylacétique cherché sous forme d'un de ses sels tels que notamment son sel de sodium, puis de déplacer ce sel par un acide minéral fort.

Le procédé de la présente invention permet d'obtenir, entre autres, les produits suivants :
- le chloro-3 hydroxy-4 phénylacétate de sodium,
- l'acide thiophène-2 acétique,
- l'acide alpha méthyl thiophène-2 acétique,
- l'acide isopropyl-2 parahydroxyphényl-2 acétique.

D'autres caractéristiques de la présente invention apparaitront à la lecture de la description suivante et des exemples donnés à titre illustratif et nullement limitatif.

Exemple 1

On chauffe une heure à 60°C puis 4 heures à 80°C une solution de :
- 1,5 mole d'orthochlorophénol,
- 0,5 mole d'acide glyoxylique à 80 % en poids dans l'eau,
- 0,5 mole d'acide phosphoreux,
- 12,5 mmoles d'acide iodhydrique à 57 %,
- 31 g d'acide acétique.

La solution est ensuite diluée avec 500 g d'eau puis elle est neutralisée à 20°C à pH = 6. Elle est ensuite soumise à un entraînement à la vapeur d'eau pour éliminer le chlorophénol non transformé puis elle est abandonnée à la cristallisation à 0°C. Le précipité obtenu est filtré, lavé à l'eau et séché à poids constant à 50°C sous vide. On obtient ainsi 0,29 mole de chloro-3 hydroxy-4 phénylacétate de sodium pur.

Exemple 2

On chauffe 2 heures au reflux une solution de:
- 7 moles de thiophène,
- 1 mole d'acide phosphoreux,
- 1 mole d'acide glyoxylique à 80 % en poids dans l'eau,
- 50 mmoles d'acide iodhydrique à 57 %,
- 420 g d'acide acétique.

La solution est ensuite refroidie puis traitée avec 5 g d'acétate de sodium anhydre et enfin concentrée sous vide. On reprend le résidu avec 100 g d'eau et 4 g de disulfite de sodium et l'on extrait l'acide cherché au toluène. Les phases organiques réunies sont lavées à l'eau, séchées et concentrées à sec sous vide. Le résidu huileux obtenu est ensuite distillé sous un vide de 0,2 mm de mercure. On obtient ainsi 0,6 mole d'acide thiophène-2 acétique pur présentant un point de fusion de 62 ± 1°C.

Exemple 3

On chauffe 15 heures au reflux une solution de:
- 5 moles de thiophène,
- 0,5 mole d'acide pyruvique,
- 0,5 mole d'acide phosphoreux,
- 25 mmoles d'acide iodhydrique à 57 %,
- 210 g d'acide acétique.

La solution obtenue est ensuite concentrée sous vide après addition de 13,2 g d'acétate de sodium anhydre. L'huile résiduelle est reprise par 350 g de diéthyle oxyde ; la phase éthérée est lavée avec une solution aqueuse saturée en bicarbonate de sodium puis à l'eau. Après élimination de l'éther sous vide, l'acide cherché est distillé sous un vide de 8 mm de mercure. On obtient ainsi 0,435 mole d'acide (thiényl-2)-2 propionique, distillant à 135 ± 2°C sous 8 mm de mercure.

Exemple 4

On chauffe 270 minutes au reflux une solution de :
- 2,5 moles de phénol,
- 0,5 mole d'acide phosphoreux,
- 0,5 mole d'acide méthyl-3-oxo-2 butanoïque,
- 25 mmoles d'acide iodhydrique à 57 %,
- 31 g d'acide acétique.

Puis la solution est concentrée sous vide après introduction de 8 g de carbonate de sodium. L'huile résiduelle est ensuite reprise avec 300 g d'eau, elle est alcalinisée à pH = 7,5 puis elle est lavée avec de l'acétate d'isopropyle. Les phases organiques réunies sont ensuite lavées avec une solution aqueuse saturée de bicarbonate de sodium. Les phases aqueuses sont réunies; on élimine sous vide les dernières traces de solvant qu'elles contiennent puis on introduit 5 g de disulfite de sodium et on les acidifie à pH = 1 à 15°C. Le précipité obtenu est filtré puis lavé à l'eau et séché sous vide à poids constant à 50°C. On isole ainsi 0,24 mole d'acide isopropyl-2-parahydroxyphényl-2 acétique pur présentant un point de fusion de 172 ± 1°C.

Il va de soi que la présente invention n'a été décrite qu'à titre purement explicatif et nullement limitatif et que toute modification utile pourra y être apportée sans sortir de son cadre.

**Revendications**

1. Procédé d'obtention d'acides acétiques substitués de formule générale I :
Ar-CHR-COOH I
dans laquelle R représente un atome d'hydrogène ou un radical alkyle en $C_1$-$C_4$ et Ar représente un

radical à caractère aromatique choisi parmi les radicaux suivants: thiényle-2, méthoxy-2 naphtyle-1, méthylènedioxy-3,4 phényle et les phényles substitués de formule générale II:

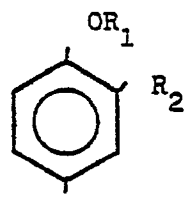

II

où $R_1$ représente un atome d'hydrogène ou un groupement alkyle en $C_1$-$C_4$ et $R_2$ représente un atome d'hydrogène, un atome d'halogène, un groupement alkyle, alcoxyle ou hydroxyle, procédé caractérisé par le fait que l'on fait réagir à chaud, en milieu acide, en phase homogène, en présence d'acide phosphoreux et de quantités catalytiques d'iode ou d'acide iodhydrique, un acide carboxylique alpha carbonylé de formule générale III :
R-CO-COOH III
dans laquelle R a la signification ci-dessus, avec un dérivé insaturé à caractère aromatique choisi parmi les suivants : thiophène, méthoxy-2 naphtalène, méthylènedioxy-1,2 benzène et les hydrocarbures aromatiques substitués de formule générale IV :

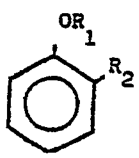

IV

dans laquelle $R_1$ et $R_2$ ont la signification ci-dessus, éventuellement au sein d'un solvant organique compatible.

2. Procédé selon la revendication 1, caractérisé par le fait que l'on fait réagir à une température supérieure à 70°C, en milieu acide, une mole d'acide carboxylique alpha carbonylé de formule générale III telle que définie à la revendication 1, avec de une à dix moles d'un dérivé insaturé à caractère aromatique tel que défini à la revendication 1, en présence d'au moins une mole d'acide phosphoreux et de 0,01 à 0,1 mole d'iode ou d'acide iodhydrique, éventuellement au sein d'acide acétique.

3. Application du procédé selon l'une quelconque des revendications 1 ou 2 à la fabrication de l'acide thiophène-2 acétique, caractérisée par le fait que le dérivé insaturé à caractère aromatique mis en oeuvre est le thiophène et que l'acide carboxylique alpha carbonylé utilisé est l'acide glyoxylique.

4. Application du procédé selon l'une quelconque des revendications 1 ou 2 à la fabrication de l'acide alpha méthyl thiophène-2 acétique, caractérisée par le fait que le dérivé insaturé à caractère aromatique mis en oeuvre est le thiophène et que l'acide carboxylique alpha carbonylé utilisé est l'acide pyruvique.

5. Application du procédé selon l'une quelconque des revendications 1 ou 2 à la fabrication de l'acide isopropyl-2-parahydroxyphényl-2 acétique caractérisée par le fait que le dérivé insaturé à caractère aromatique mis en oeuvre est le phénol et que l'acide carboxylique alpha carbonylé utilisé est l'acide méthyl-3 oxo-2 butanoïque.

6. Application du procédé selon l'une quelconque des revendications 1 ou 2 à la fabrication de l'acide chloro-3 hydroxy-4 phénylacétique caractérisée par le fait que le dérivé insaturé à caractère aromatique mis en oeuvre est l'orthochlorophénol et que l'acide carboxylique alpha carbonylé utilisé est l'acide glyoxylique.

## Patentansprüche

1. Verfahren zur Herstellung von substituierten Essigsäuren der allgemeinen Formel (I)
Ar – CHR – COOH (I),
worin R ein Wasserstoffatom oder eine $C_1$-$C_4$-Alkylgruppe bedeutet und Ar einen Rest mit aromatischem Charakter ausgewählt aus folgenden Resten: 2-Thienyl, 2-Methoxy-1-naphthyl, 3,4-Methylendioxyphenyl und substituiertem Phenyl der allgemeinen Formel

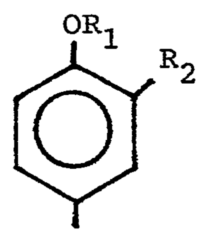

(II)

darstellt, wobei $R_1$ ein Wasserstoffatom oder eine $C_1$–$C_4$ Alkylgruppe ist und $R_2$ ein Wasserstoffatom, ein Halogenatom oder eine Alkyl-, Alkoxy- oder Hydroxylgruppe bedeutet, dadurch gekennzeichnet, daß man eine α-Carbonylcarbonsäure der allgemeinen Formel (III)
R – CO – COOH (III),
worin R die oben angegebene Bedeutung hat, mit einem ungesättigten Derivat mit aromatischem Charakter ausgewählt aus Thiophen, 2-Methoxynaphthalin, 1,2-Methylendioxybenzol und substituierten aromatischen Kohlenwasserstoffen der allgemeinen Formel (IV)

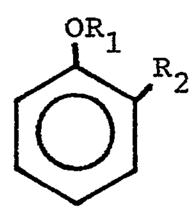

(IV),

worin $R_1$ und $R_2$ die oben angegebene Bedeutung haben, gegebenenfalls in einem verträglichen organischen Lösungsmittel unter Erhitzen in einem sauren Medium in homogener Phase in Anwesenheit von phosphoriger Säure und katalytischer Mengen von Jod oder Jodwasserstoffsäure umsetzt.

2. Verfahren nach Anspruch 1, dadurch gekennzeichnet, daß man bei einer Temperatur oberhalb 70°C in saurem Medium 1 Mol α-Carbonylcarbonsäure der allgemeinen Formel (III), wie in Anspruch 1 definiert, mit 1 bis 10 Mol eines ungesättigten Derivates mit aromatischem Charakter, wie in Anspruch 1 definiert, in Anwesenheit von mindestens 1 Mol phosphoriger Säure und 0,01 bis 0,1 Mol Jod oder Jodwasserstoffsäure, gegebenenfalls in Essigsäure, umsetzt.

3. Anwendung des Verfahrens nach einem der Ansprüche 1 oder 2 für die Herstellung von 2-Thiophenessigsäure, dadurch gekennzeichnet, daß das verwendete ungesättigte Derivat mit aromatischem Charakter Thiophen ist und die verwendete α-Carbonylcarbonsäure Glyoxylsäure ist.

4. Anwendung des Verfahrens nach einem der Ansprüche 1 oder 2 für die Herstellung von α-Methyl-2-thiophenessigsäure, dadurch gekennzeichnet, daß das verwendete ungesättigte Derivat mit aromatischem Charakter Thiophen ist und die verwendete Q-Carbonylcarbonsäure Pyruvinsäure ist.

5. Anwendung des Verfahrens nach einem der Ansprüche 1 oder 2 für die Herstellung von 2-Isopropyl-2-p-hydroxyphenylessigsäure, dadurch gekennzeichnet, daß das verwendete ungesättigte Derivat mit aromatischem Charakter Phenol ist und die verwendete Q-Carbonylcarbonsäure 3-Methyl-2-oxobutansäure ist.

6. Anwendung des Verfahrens nach einem der Ansprüche 1 oder 2 für die Herstellung von 3-Chlor-4-hydroxyphenylessigsäure, dadurch gekennzeichnet, daß das verwendete ungesättigte Derivat mit aromatischem Charakter o-Chlorphenol ist und die verwendete α-Carbonylcarbonsäure Glyoxylsäure ist.

**Claims**

1. A process for the preparation of substituted acetic acids having the general Formula I:

Ar–CHR–COOH I

where R denotes a hydrogen atom or a $C_1$–$C_4$ alkyl radical and Ar denotes a radical of aromatic nature selected from the following radicals: 2-thienyl, 2-methoxy-1-naphthyl, 3,4-methylenedioxy phenyl and substituted phenyls having the general Formula II:

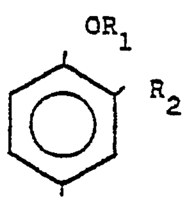

II

where $R_1$ denotes a hydrogen atom or a $C_1$–$C_4$ alkyl group and $R_2$ denotes a hydrogen atom, a halogen atom, an alkyl, alkoxyl or hydroxyl group, the process being characterized in that a carbonylated alpha carboxylic acid having the general Formula III:

R–CO–COOH III

where R has the above meaning, is reacted hot in homogeneous phase in an acid medium in the presence of phosphoric acid and catalytic quantities of iodine and hydriodic acid with an unsaturated derivative of aromatic nature selected from the following: thiophene, 2-methoxy naphthalene, 1,2-methylenedioxy benzene and substituted aromatic hydrocarbons having the general Formula IV:

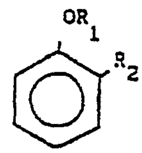

IV

where $R_1$ and $R_2$ have the above meanings, if necessary in a compatible organic solvent.

2. A process according to claim 1, characterized in that one mole of carbonylated alpha carboxylic acid having the general Formula III as defined in Claim 1 is reacted at a temperature higher than 70°C in an acid medium with between one and ten moles of an unsaturated derivative of aromatic nature as defined in Claim 1, in the presence of at least one mole of phosphoric acid and 0.01 to 0.1 mole of iodine or hydriodic acid, if necessary in acetic acid.

3. Application of the process according to either of claims 1 or 2 to the preparation of 2-thiophene acetic acid, characterized in that the unsaturated derivative of aromatic nature used is thiophene and the carbonylated alpha carboxylic acid used is glyoxylic acid.

4. Application of the process according to either of claims 1 or 2 to the preparation of alpha 2-thiophene methyl acetic acid, characterized in that the unsaturated derivative of aromatic nature used is thiophene and the carbonylated alpha carboxylic acid used is pyruvic acid.

5. Application of the process according to either of claims 1 or 2 to the preparation of 2-isopropyl-2-parahydroxy phenyl acetic acid, characterized in that the unsaturated derivative of aromatic nature used is phenyl and the carbonylated alpha carboxylic acid used is 3-methyl-2-oxobutanoic acid.

6. Application of the process according to either of claims 1 or 2 to the preparation of 3-chloro-4-hydroxy phenyl acetic acid, characterized in that the unsaturated derivative of aromatic nature used is orthochloro phenol and the carbonylated alpha carboxylic acid used is glyoxylic acid.